# EUROPEAN PATENT SPECIFICATION

(11) **EP 3 401 410 B1**
(45) Date of publication and mention of the grant of the patent: **30.12.2020**
(21) Application number: 18175599.2
(22) Date of filing: 26.06.2011
(51) Int. Cl.: C13K 1/02, B01D 53/14, C01B 7/07, C12P 7/08, C12P 7/10, C13B 5/04, C13B 10/02, C13K 11/00, C13K 13/00, C12P 19/02, C12P 19/14

(54) **METHODS FOR PRODUCTION OF SUGAR MIXTURES**
VERFAHREN ZUR HERSTELLUNG VON ZUCKERMISCHUNGEN
MÉTHODES DE PRODUCTION DE MÉLANGES DE SUCRES

(30) Priority: 26.06.2010 US 35889410 P; 08.07.2010 IL 20689610; 29.07.2010 IL 20731310; 06.02.2011 WO PCT/IL2011/000130; 30.05.2011 US 201161491243 P; 23.06.2011 US 201161500169 P
(43) Date of publication of application: 14.11.2018
(60) Divisional application: 20208564.3
(62) Document number(s) of the earlier application(s) in accordance with Art. 76 EPC: 11797729.8 / 2 585 606
(73) Proprietor: Virdia, Inc., Raceland, LA 70394 (US)
(72) Inventor: EYAL, Aharon, 93629 Jerusalem (IL); JANSEN, Robert, 2685-202 Portela LRS (PT); VITNER, Asher, 96920 Jerusalem (IL); MALI, Revital, 96781 Jerusalem (IL)
(74) Representative: J A Kemp LLP

(56) References cited:
- WO-A1-2010/009515
- US-A- 4 237 110

## Description

### FIELD OF THE INVENTION

This invention relates to sugars and production and use thereof.

### BACKGROUND OF THE INVENTION

The carbohydrate-conversion industry is large and rapidly increasing in size. Currently, about 100 million tons of carbohydrates are fermented annually, primarily to provide fuel-grade ethanol. This number is predicted to triple in the next decade.

Millions of tons of carbohydrates are also fermented every year to provide food and feed products, such as citric acid and lysine. Also large and increasing is fermentation to produce other products, such as monomers for the polymer industry, e.g. lactic acid for the production of polylactide.

Fermentation media typically include, in addition to carbohydrates and/or another carbon source, other nutrients and factors such as nitrogen sources, minerals, vitamins, and growth factors. In some cases, fermentation media comprise well identified chemicals. In other cases, various preparations (e.g. yeast extract or tryptone broth) are incorporated without fully understanding the effect of each component in the preparation. Some of those preparations result from natural sources, such as extracts. Some of those preparations are of relatively high cost.

With the advent of molecular biology techniques, a new generation of industrial fermentation, also known as conversion, based upon genetically modified microorganisms has emerged. In some cases these microorganisms rely upon inducible promoters for induction of a specific gene. Some of the inducible promoters respond to specific sugars.

Although conversion of lignocellulosic material to carbohydrates via enzyme-catalyzed and/or acid-catalyzed hydrolysis of polysaccharides and pyrolysis of lignocellulosic material have been previously described, industrial scale application of the proposed technologies has presented technical problems which remain to be overcome. Hydrolysis of hemicellulose is relatively easy, but hydrolysis of cellulose (typically more than 50% of total polysaccharides) is more difficult due to its partial crystalline structure.

This application refers to various solvents defined in terms of Hoy's cohesion parameter Delta-P and/or Delta-H. By way of review:
Delta-P is the polarity related component of Hoy's cohesion parameter and delta-H is the hydrogen bonding related component of Hoy's cohesion parameter.

The cohesion parameter, as referred to above or, solubility parameter, was defined by Hildebrand as the square root of the cohesive energy density: $δ = \sqrt{\frac{Δ {E}_{vap}}{V}}$ where ΔEᵥₐₚ and V are the energy or heat of vaporization and molar volume of the liquid, respectively. Hansen extended the original Hildebrand parameter to a three-dimensional cohesion parameter. According to this concept, the total solubility parameter, delta, is separated into three different components, or, partial solubility parameters relating to the specific intermolecular interactions: ${δ}^{2} = {δ}_{d}^{2} + {δ}_{p}^{2} + {δ}_{h}^{2}$ in which delta-D, delta-P and delta-H are the dispersion, polarity, and Hydrogen bonding components, respectively. Hoy proposed a system to estimate total and partial solubility parameters. The unit used for those parameters is MPa^{1/2}. A detailed explanation of that parameter and its components can be found in "CRC Handbook of Solubility Parameters and Other Cohesion Parameters", second edition, pages 122-138 . That and other references provide tables with the parameters for many compounds. In addition, methods for calculating those parameters are provided.

US4237110 describes a process in which a crude product obtained from the acid hydrolysis of cellulose, the product comprising concentrated hydrochloric acid and the various sugars obtained from the acid hydrolysis of cellulose, is separated into a first fraction comprising concentrated hydrochloric acid and a second fraction comprising the sugars by a process comprising: (1) contacting the crude product with an organic solvent consisting essentially of at least one C5-C9 alcohol such that the organic solvent is enriched with the concentrated hydrochloric acid, (2) separating the enriched organic solvent from the concentrated hydrochloric acid depleted crude product, and (3) recovering the concentrated hydrochloric acid from the enriched organic solvent.

WO2010009515 describes a method for hydrolysing a polysaccharide, the method comprising the steps of: (i) contacting the polysaccharide with mesophilic and thermophilic hydrolytic enzymes to produce a reaction mixture, (ii) incubating the reaction mixture at a temperature suitable for activity of the mesophilic hydrolytic enzymes, and (iii) incubating the reaction mixture at a temperature suitable for activity of the thermophilic hydrolytic enzymes, wherein the polysaccharide is hydrolysed by said mesophilic and thermophilic hydrolytic enzymes to produce two or more polysaccharide fragments.

### SUMMARY OF THE INVENTION

One aspect of the disclosure relates to heterogeneous sugar mixtures. The term "sugar" as used in this specification and the accompanying claims refers to monosaccharides and oligosaccharides (disaccharides or greater) soluble in water at 25 degrees centigrade. Throughout this application, the term disaccharide refers to sugar dimers and "higher oligosaccharides" or "higher saccharide" refers to oligomers comprising three or more sugar units. According to various exemplary aspects of the disclosure dimers may be homo-dimers and/or hetero-dimers. Alternatively or additionally, higher oligosaccharides may include same and/or different sugar units.

In many exemplary aspects of the disclosure, the sugar mixtures result from acid hydrolysis of lignocellulosic or "woody" substrates. In some exemplary aspects, acid hydrolysis is conducted with HCl, optionally at a concentration of 37% W/W HCl/ [HCl+water], optionally 39, 41, 43 or even 45 % HCl on the same basis. In some exemplary aspects of the disclosure, the hydrolysis is conducted at temperatures below 50 degrees centigrade, optionally below 40 degrees, optionally below 30 degrees, optionally at 25 degrees or less. In some exemplary aspects, at least a portion of the hydrolysis is conducted at 20 degrees or less, optionally 15 degrees or less.

According to various exemplary aspects the sugar mixture is provided as a syrup and/or liquid, optionally including residual acid from hydrolysis. Total sugar concentrations in such a syrup/liquid can be 15, 20, 25, 30, 35, 40, 45 or 50% by weight or intermediate or higher percentages. Optionally, the mixture is provided as dry crystals.

In some exemplary aspects, a percentage of oligosaccharides to total saccharides in the mixture is greater than 4, optionally 6, optionally 8, optionally 10, optionally 15% or intervening or greater percentages.

In some exemplary aspects, a percentage of disaccharides to total saccharides in the mixture is greater than 3, optionally 5, optionally 7, optionally 10% or intervening or greater percentages.

"Disaccharide" indicates two sugars connected by an alpha bond or by a beta bonds or bonded via various hydroxyls on the molecule, and combinations thereof.

In some exemplary aspects, a percentage of pentoses to total saccharides in the mixture is greater than 3, optionally 5, optionally 7, optionally 10% or intervening or greater percentages. Optionally, at least a portion of the pentose is present as part of a disaccharide or longer oligosaccharide.

According to various exemplary aspects the mixture includes at least one alpha-bonded di-glucose and/or at least one beta-bonded di-glucose. Optionally, at least a portion of the di-glucoses are present as parts of higher oligosaccharides.

In some exemplary aspects, residual acid (e.g. HCl) may be present in the mixture. Various exemplary aspects of the disclosure are concerned with ways to remove this residual acid. Optionally, such removal contributes to added value for use in downstream processes (e.g. fermentation). In some exemplary aspects, removal of residual acid involves extraction with an extractant containing an alcohol. Optionally, two or more extractions are conducted. In some exemplary aspects, at least one of the extractions employs a mixture of two solvent types.

Another aspect of the disclosure relates to hydrolyzing a lignocellulosic substrate in HCl to form a hydrolyzate comprising total saccharides to (total saccharides + water) of at least 20%, optionally 25%, optionally 30% by weight and de-acidifying the hydrolyzate while increasing the sugar concentration. Optionally, the sugar concentration is increased to 35, 40, 45 or 50% or greater or intermediate percentages. In some exemplary aspects, the disaccharides in the de-acidified hydrolyzate are at least 5%, optionally 10%, optionally 20%, optionally 30% of the total saccharides or intermediate or greater percentages. Optionally, a portion of the saccharides in the de-acidified hydrolyzate can be enzymatically digested.

Another aspect of the disclosure relates to fermenting such a sugar mixture in a fermentor to produce a desired fermentation product or "conversion product". Optionally, the fermentation product can include one or more of alcohols, carboxylic acids, amino acids, monomers for production of industrially important polymers and proteins. In some exemplary aspects, a fermentation product is produced and then converted into the monomer (e.g. 3-hydroxy-propionic acid to be converted into acrylic acid, which is then polymerized). In other exemplary aspects, the monomer is produced directly (e.g. lactic acid as a source of polylactide).

Optionally, the proteins are heterologous proteins produced by genetically modified microorganisms. Such heterologous proteins include, but are not limited to, hormones, enzymes (e.g. cellulases), growth factors, cytokines and antibodies. Optionally, the antibodies are fusion proteins including a non-immunoglobulin domain.

An additional aspect of the disclosure relates to enzymatic hydrolysis of a portion of the sugars in the mixture. For purposes of this specification and the accompanying claims, the term "enzyme" indicates a single enzyme or a mixture including two or more enzymes. Optionally, an enzyme is provided as a crude preparation (e.g. cell extract) characterized by a type and/or level of activity, as opposed to a precise molecular definition. According to various exemplary aspects enzymes capable of hydrolyzing alpha and/or beta bonds are used. Optionally, specificity for a desired bond type can be achieved by appropriate enzyme selection and/or selection of suitable reaction conditions. In some exemplary aspects at least 10% of disaccharides in the mixture are converted to monosaccharides by this enzymatic treatment. Alternatively or additionally, at least 10% of oligosaccharides in the mixture are enzymatically hydrolyzed to release additional monosaccharides. In some exemplary aspects, enzymes are immobilized. Optionally, immobilization can be on beads and/or a membrane. In some exemplary aspects, immobilization contributes to an increase in yield of an enzymatic hydrolysis product per unit of enzyme.

For purposes of this specification and the accompanying claims, and where not further defined, an "S1 solvent" or "S1" is an organic solvent with a water solubility of less than 15% characterized by a polarity related component of Hoy's cohesion parameter (delta-P) between 5 and 10 MPa^{1/2} and/or by a hydrogen bonding related component of Hoy's cohesion parameter (delta-H) between 5 and 20 MPa^{1/2}. In some exemplary aspects of the disclosure, HCl tends to selectively transfer to an S1 solvent upon contact therewith.

For purposes of this specification and the accompanying claims, and where not further defined, an "S2 solvent" or "S2" is an organic solvent having a water solubility of at least 30% and characterized by a delta-P greater than 8 MPa^{1/2} and/or a delta-H greater than 12 MPa^{1/2}. In some exemplary aspects of the disclosure, HCl tends to selectively transfer to an extractant including both S1 and S2 solvents upon contact therewith.

For purposes of this specification and the accompanying claims "extract" and "extraction" indicate bringing an extractant into contact with a substrate and then separating an extract from an extracted substrate.

According to various exemplary aspects of the disclosure an extraction may be on an indicate stream or fraction *per se* or on a modified stream or fraction. Optional modifications include, but are not limited to, dilution, concentration, mixing with another stream or fraction, temperature adjustment, and filtration. Optionally, two or more modifications may be performed prior to extraction.

"Woody materials" or "lignocellulosic materials" are an attractive and environment-friendly substrate for sugar production since they are obtained from renewable resources. Many non-food lignocellulosic materials are potential sources of soluble carbohydrates. These lignocellulosic materials include, but are not limited to, wood and by-products of wood processing (e.g. chips, sawdust, and shavings) as well as residual plant material from agricultural products and paper industry byproducts (e.g. cellulose containing residues and/or paper pulp)

Residual plant material from agricultural products includes processing by-products and field remains.

Processing by-products include, but are not limited to, corn cobs, sugar cane bagasse, sugar beet pulp, empty fruit bunches from palm oil production, straw (e.g. wheat or rice), soy bean hulls, residual meals from the vegetable oil industry (e.g. soybean, peanut, corn or rapeseed), wheat bran and fermentation residue from the beer and wine industries.

Field remains include, but are not limited to, corn stover, post harvest cotton plants, post harvest soybean bushes and post harvest rapeseed plants.

Lignocellulosic materials also include "energy crops" such as switch grass and broom grass which grow rapidly and generate low-cost biomass specifically as a source of carbohydrates.

These lignocellulosic carbohydrate sources contain cellulose, hemicellulose and lignin as their main components and also contain mineral salts (ashes) and lipophilic organic compounds, such as tall oils. The degree and type of these non-carbohydrate materials can create technical problems in production of soluble carbohydrates.

Lignocellulosic materials typically contain 65-80% cellulose and hemicelluloses on a dry matter basis. Cellulose and hemicellulose are polysaccharides which can release carbohydrates suitable for fermentation and/or chemical conversion to products of interest if they are hydrolyzed. Lignin is typically resistant to acid hydrolysis.

It will be appreciated that the various aspects described above relate to solution of technical problems associated with obtaining specific ratios of disaccharides and/or higher oligomers relative to total saccharides produced by acid hydrolysis of a lignocellulosic substrate.

Alternatively or additionally, it will be appreciated that the various aspects described above relate to solution of technical problems associated with the need for defined mixtures of sugars containing specific disaccharides and/or higher oligomers. In many cases this "need" is defined by a specific downstream application.

In some exemplary aspects of the disclosure, there is provided a sugar mixture including: (i) monosaccharides; (ii) oligosaccharides in a ratio to total saccharides ≥ 0.06; (iii) disaccharides in a ratio to total saccharides ≥ 0.05; (iv) pentose in a ratio to total saccharides ≥ 0.05; (v) at least one alpha-bonded di-glucose; and (vi) at least one beta-bonded di-glucose.

Optionally, the mixture has a higher oligosaccharides in a ratio to total saccharides ≤ 0.2.

Optionally, the mixture has a ratio of at least one of the alpha-bonded di-glucose and the beta-bonded di-glucose relative to total saccharides is ≥ 0.01.

Optionally, the mixture has a ratio of at least one of the alpha-bonded di-glucose and the beta-bonded di-glucose relative to total saccharides is ≥ 0.03.

Optionally, the alpha-bonded di-glucose includes at least one member of the group consisting of maltose, isomaltose and trehalose.

Optionally, the beta-bonded di-glucose includes at least one member selected from the group consisting of gentiobiose, sophorose and cellobiose.

In some exemplary aspects of the disclosure, there is provided a method including (a) hydrolyzing a lignocellulosic material in a medium containing HCl in a ratio to (HCl + water) ≥ 0.37 to form a hydrolyzate including total saccharides in a ratio to (total saccharides + water) ≥ 0.20 by weight; (b) de-acidifying the hydrolyzate to form a de-acidified hydrolyzate including: (i) total saccharides in a ratio to (total saccharides + water) ≥ 0.35 and; (ii) total disaccharides in a ratio to total saccharides ≥ 0.05; and (c) adjusting a composition of the de-acidified hydrolyzate to form a mixture according to any of claims 1 to 6.

In some exemplary aspects of the disclosure, there is provided a method including (a) hydrolyzing a lignocellulosic material in a medium containing HCl in a ratio to (HCl + water) ≥ 0.37 by weight to form a hydrolyzate including total saccharides in a ratio to (total saccharides + water) ≥ 0.20 by weight; (b) de-acidifying the hydrolyzate to form a de-acidified hydrolyzate including a mixture as described above.

Optionally, the hydrolyzing is conducted in a counter-current mode of operation.

Optionally, the hydrolyzing is conducted at a temperature of less than 25°C.

Optionally, the lignocellulosic material includes softwood, for example, pine.

Optionally, the de-acidifying includes selective extraction of HCl and water with an extractant including alcohol.

Optionally, the de-acidifying is conducted at a temperature of less than 80°C.

In some exemplary aspects of the disclosure, there is provided a method including: (i) providing a preparation including HCl and a sugar mixture according to any of claims 1 to 6, and (ii) de-acidifying the preparation to form a de-acidified preparation.

Optionally, the de-acidifying includes selective extraction of HCl with an extractant including alcohol.

Optionally, the de-acidifying is conducted at a temperature of less than 80°C.

In some exemplary aspects of the disclosure, there is provided a method including: (a) providing a fermentor; and (b) fermenting a medium including a sugar mixture as described above in the fermentor to produce a fermentation product.

In some exemplary aspects of the disclosure, there is provided a method including: (a) providing a fermentor; and (b) fermenting a medium including a de-acidified hydrolyzate according as described above or a de-acidified preparation as described above to produce a fermentation product.

Optionally, the fermentation product includes at least one member selected from the group consisting of alcohols, carboxylic acids, amino acids, monomers for the polymer industry and proteins.

Optionally, the method includes processing the fermentation product to produce a consumer product selected from the group consisting of detergent, polyethylene-based products, polypropylene-based products, polyolefin-based products, polylactic acid (polylactide)- based products, polyhydroxyalkanoate-based products and polyacrylic-based products.

Optionally, the detergent includes a sugar-based surfactant, a fatty acid-based surfactant, a fatty alcohol-based surfactant, or a cell-culture derived enzyme.

Optionally, the polyacrylic-based product is selected from plastics, floor polishes, carpets, paints, coatings, adhesives, dispersions, flocculants, elastomers, acrylic glass, absorbent articles, incontinence pads, sanitary napkins, feminine hygene products, and diapers.

Optionally, the polyolefin-based products are selected from milk jugs, detergent bottles, margarine tubs, garbage containers, water pipes, absorbent articles, diapers, non wovens , HDPE toys and HDPE detergent packagings.

Optionally, the polypropylene based products are selected from absorbent articles, diapers and non wovens.

Optionally, the polylactic acid based products are selected from packaging of agriculture products and of dairy products, plastic bottles, biodegradable products and disposables.

Optionally, the polyhydroxyalkanoate based products are selected from packaging of agriculture products, plastic bottles, coated papers, molded or extruded articles, feminine hygiene products, tampon applicators, absorbent articles, disposable nonwovens and wipes, medical surgical garments, adhesives, elastometers, films, coatings, aqueous dispersants, fibers, intermediates of pharmaceuticals and binders.

Optionally, the fermentation product includes at least one member of the group consisting of ethanol, butanol, isobutanol, a fatty acid, a fatty acid ester, a fatty alcohol and biodiesel.

Optionally, the method includes processing of the fermentation product to produce at least one product selected from the group consisting of an isobutene condensation product, jet fuel, gasoline, gasohol, diesel fuel, drop-in fuel, diesel fuel additive, and a precursor thereof.

Optionally, the gasohol is ethanol-enriched gasoline or butanol-enriched gasoline.

Optionally, the product is selected from the group consisting of diesel fuel, gasoline, jet fuel and drop-in fuels.

In some exemplary aspects of the disclosure, there is provided a consumer product, a precursor of a consumer product, or an ingredient of a consumer product produced from a fermentation product as described above.

In some exemplary aspects of the disclosure, there is provided a consumer product, a precursor of a consumer product, or an ingredient of a consumer product including at least one fermentation product produced by a method as described above, wherein the fermentation product is selected from carboxylic and fatty acids, dicarboxylic acids, hydroxylcarboxylic acids, hydroxyl di-carboxylic acids, hydroxyl-fatty acids, methylglyoxal, mono-, di-, or poly-alcohols, alkanes, alkenes, aromatics, aldehydes, ketones, esters, biopolymers, proteins, peptides, amino acids, vitamins, antibiotics, and pharmaceuticals.

Optionally, the product is ethanol-enriched gasoline, jet fuel, or biodiesel.

In some exemplary aspects of the disclosure, there is provided consumer product, a precursor of a consumer product, or an ingredient of a consumer product according as described above, wherein the consumer product has a ratio of carbon-14 to carbon-12 of about 2.0 x 10⁻¹³ or greater.

Optionally, the consumer product including an ingredient according as described above and an additional ingredient produced from a raw material other than lignocellulosic material.

Optionally, the ingredient and the additional ingredient produced from a raw material other than lignocellulosic material are essentially of the same chemical composition.

Optionally, the consumable product includes a marker molecule at a concentration of at least 100ppb.

Optionally, the marker molecule is selected from the group consisting of furfural, hydroxy-methyl furfural, products of furfural or hydroxy-methylfurfural condensation, color compounds derived from sugar caramelization, levulinic acid, acetic acid, methanol, galcturonic acid, and glycerol.

In some exemplary aspects of the disclosure, there is provided a method including: (a) de-acidifying an acid hydrolyzate including total saccharides in a ratio to (total saccharides + water) ≥ 0.20 by weight to produce a sugar mixture with total saccharides in a ratio to (total saccharides + water) ratio ≥ 0.35; the mixture including monosaccharides, the mixture having disaccharides in a ratio to total saccharides ≥ 0.05; and (b) enzymatically hydrolyzing the mixture with an enzyme capable of catalyzing hydrolysis of alpha bonds in the mixture so that at least 10% of the disaccharides are converted to monosaccharides; and (c) converting at least a portion of the saccharides to a conversion product.

Optionally, the sugar mixture includes higher oligosaccharides.

Optionally, at least 10% of the higher oligosaccharides are hydrolyzed.

Optionally, the acid hydrolyzate is the result of counter-current hydrolysis.

Optionally, the acid hydrolyzate is the result of hydrolysis conducted at a temperature of less than 25°C.

Optionally, the de-acidifying includes extraction with an extractant including an alcohol.

Optionally, the de-acidifying is conducted at a temperature of less than 80°C.

Optionally, the enzymatically hydrolyzing includes use of an enzyme capable of catalyzing hydrolysis of beta bonds.

Optionally, the enzyme includes at least one enzyme selected from the group consisting of amylases cellulases, hemicellulases, transglucosidases, glucoamylases, alpha-glucosidases and pullulanases.

Optionally, the enzymatically hydrolyzing includes use of an immobilized enzyme.

Optionally, at least a portion of the converting is conducted simultaneously with the enzymatically hydrolyzing.

Optionally, the total saccharides ratio to (total saccharides + water) is ≥ 0.15 during the enzymatically hydrolyzing.

Optionally, the enzymatically hydrolyzing includes incubation of the mixture with a microorganism.

Optionally, the converting includes fermentation.

Optionally, the sugar mixture includes at least one pentose in a ratio to total saccharides ≥ 0.05.

Optionally, the de-acidifying includes extracting the hydrolyzate, with a first extractant including an S1 solvent to form an HCl-carrying first extract and an HCl-depleted sugar solution.

Optionally, the de-acidifying includes including chromatographically separating the HCl-depleted sugar solution to produce a monosaccharide enriched monomer cut and an acid cut enriched in disaccharides and higher oligosaccharides.

Optionally, the de-acidifying includes subsequently extracting the HCl-depleted sugar solution with a second extractant including S1 and a second solvent (S2).

Optionally, the S1 of the extracting and the subsequently extracting each independently include at least one member selected from the group consisting of alcohols, ketones and aldehydes having at least 5 carbon atoms and combinations thereof.

Optionally, the second extractant is characterized by at least one of:
a delta-P greater than the delta-P of the first extractant by at least 0.2 MPa^{1/2} ; and
a delta-H greater than the delta-H of the first extractant by at least 0.2 MPa^{1/2}.

Optionally, S2 includes at least one member selected from the group consisting of C1-C4 mono- or poly-alcohols, aldehydes and ketones.

Optionally, a ratio of HCl to total saccharides in the sugar mixture is ≤ 0.03 by weight.

Unless otherwise defined, all technical and scientific terms used herein have the same meaning as commonly understood by one of ordinary skill in the art to which this invention belongs. As used herein, the terms "comprising" and "including" or grammatical variants thereof are to be taken as specifying inclusion of the stated features, integers, actions, ratios or components without precluding the addition of one or more additional features, integers, actions, ratios, components or groups thereof. This term is broader than, and includes the terms "consisting of' and "consisting essentially of' as defined by the Manual of Patent Examination Procedure of the United States Patent and Trademark Office.

The term "method" refers to manners, means, techniques and procedures for accomplishing a given task including, but not limited to, those manners, means, techniques and procedures either known to, or readily developed from known manners, means, techniques and procedures by practitioners of architecture and/or computer science.

Percentages (%) and/or ratios of sugars (saccharides) to a total mixture, as well as ratios of various sugars to one another (e.g. monosaccharides to disaccharides) are W/W (weight per weight) unless otherwise indicated. Percentages (%) and/or ratios of HCl are also expressed as W/W (weight per weight) unless otherwise indicate.

Specifically, the present invention provides the following:
[1] A method comprising:
   (a) hydrolyzing a lignocellulosic material in a medium containing HCl in a ratio to (HCl + water) ≥ 0.37 by weight to form a hydrolyzate comprising total saccharides in a ratio to (total saccharides + water) ≥ 0.20 by weight; and
   (c) de-acidifying said hydrolyzate to form a de-acidified hydrolyzate comprising: (i) monosaccharides, wherein the monosaccharides comprise a pentose in a ratio to total saccharides ≥ 0.05; and (ii) oligosaccharides in a ratio to total saccharides ≥ 0.06, wherein the oligosaccharides comprise disaccharides in a ratio to total saccharides ≥ 0.05, wherein the disaccharides comprise at least one alpha-bonded di-glucose and at least one beta-bonded di-glucose;
   wherein said de-acidifying comprises (i) extracting the hydrolysate with a first extractant including an S1 solvent to form an HCl-carrying first extract and an HCl-depleted sugar solution, and further comprises removing residual HCl in said HCl-depleted sugar solution by at least one of (ii-a) chromatographic separation and (ii-b) extraction with a second extractant including S1 and a second solvent S2;
   wherein the S1 solvent is an organic solvent with a water solubility of less than 15% and characterised by a polarity related component of Hoy's cohesion parameter (delta-P) between 5 and 10 MPa^{1/2} and/or by a hydrogen bonding related component of Hoy's cohesion parameter (delta-H) between 5 and 20 MPa^{1/2}, and which is a solvent selected from the group consisting of alcohols, ketones and aldehydes having at least 5 carbon atoms; and
   wherein the S2 solvent is an organic solvent with a water solubility of at least 30% and characterised by a delta-P greater than 8 MPa^{1/2} and/or a delta-H greater than 12 MPa^{1/2}, and which is a solvent selected from the group consisting of C1-C4 mono- or poly-alcohols, aldehydes and ketones.
[2] The method according to [1], wherein the de-acidified hydrolyzate comprises total saccharides in a ratio to (total saccharides + water) ≥ 0.35.
[3] The method according to [1] or [2], wherein the de-acidified hydrolyzate has a ratio of HCl to total saccharides of ≤ 0.03 by weight.
[4] The method according to any one of [1] to [3], wherein the de-acidified hydrolyzate has higher oligosaccharides in a ratio to total saccharides ≤ 0.2.
[5] The method according to [1] to [4], wherein a ratio of at least one of said alpha- bonded di-glucose and said beta-bonded di-glucose relative to total saccharides is (i) > 0.01 in the de-acidified hydrolyzate or (ii) > 0.03 in the de-acidified hydrolyzate.
[6] The method according to [1] to [5], wherein said alpha-bonded di-glucose includes at least one of maltose, isomaltose and trehalose.
[7] The method according to [1] to [6], wherein said beta-bonded di-glucose includes at least one of gentiobiose, sophorose and cellobiose.
[8] The method according to [1] to [7], wherein the S1 solvent is hexanol or 2-ethyl hexanol.
[9] The method according to [1] to [8], which comprises removing residual HCl in the HCl-depleted sugar solution by (ii-a) chromatographic separation.
[10] The method according to [1] to [9], where said hydrolyzing step (a) forms said hydrolyzate and residual lignin, and said method further comprises:
   (b) separating said hydrolyzate from said residual lignin before said de-acidifying step (c).
[11] The method according to [1] to [10], which further comprises
   (d) enzymatically hydrolyzing the de-acidified hydrolyzate with an enzyme capable of catalyzing hydrolysis of alpha bonds in the mixture so that at least 10% of the disaccharides are converted to monosaccharides.
[12] The method according to [1] to [11], which further comprises
   (e) providing a fermentor; and
   (f) fermenting a medium comprising the de-acidified hydrolyzate in said fermentor to produce a fermentation product.
[13] The method according to [12], wherein said method further comprises:
   (g1) processing said fermentation product to produce a consumer product selected from the group consisting of detergent, polyethylene-based products, polypropylene-based products, polyolefin-based products, polylactic acid (polylactide)-based products, polyhydroxyalkanoate-based products and polyacrylic-based products; or
   (g2) producing a consumer product comprising the fermentation product, wherein said fermentation product is at least one selected from carboxylic and fatty acids, dicarboxylic acids, hydroxylcarboxylic acids, hydroxyl di-carboxylic acids, hydroxyl-fatty acids, methylglyoxal, mono-, di-, or poly-alcohols, alkanes, alkenes, aromatics, aldehydes, ketones, esters, biopolymers, proteins, peptides, amino acids, vitamins, antibiotics, and pharmaceuticals; or
   (g3) producing a consumer product comprising the fermentation product, wherein said consumer product is ethanol-enriched gasoline, jet fuel, or biodiesel.

### BRIEF DESCRIPTION OF THE DRAWINGS

In order to understand the invention and to see how it may be carried out in practice, embodiments will now be described, by way of non-limiting example only, with reference to the accompanying figures. In the figures, identical and similar structures, elements or parts thereof that appear in more than one figure are generally labeled with the same or similar references in the figures in which they appear. Dimensions of components and features shown in the figures are chosen primarily for convenience and clarity of presentation and are not necessarily to scale. The attached figures are:
**Fig. 1** is a schematic overview of a system illustrating the industrial context of some exemplary aspects of the disclosure;
**Figs. 2a** and **2b** are each simplified flow diagrams of methods according to exemplary aspects of the disclosure;
**Fig. 3** is a simplified flow diagram of a method according to an exemplary aspect of the disclosure;
**Figs. 4a** and **4b** are each simplified flow diagrams of methods according to exemplary aspects of the disclosure;
**Fig. 5** is a simplified flow diagram of a method according to an exemplary aspect of the disclosure; and
**Fig. 6** is a simplified flow diagram of a portion of the method depicted in Fig. 5 in greater detail according to an exemplary aspect of the disclosure.

### DETAILED DESCRIPTION

Aspects of the disclosure relate to sugar mixtures, their preparation and use. Specifically, some aspects of the disclosure can be used as substrates for microbial fermentation. Optionally, the specific microorganisms employed are selected to utilize one or more sugars present in the mixture. Alternatively or additionally, some aspects of the disclosure relate to adjusting one or more component ratios within a mixture to render the mixture more valuable for a specific downstream application.

The principles and operation of methods according to exemplary aspects of the disclosure may be better understood with reference to the drawings and accompanying descriptions.

Before explaining at least one aspect of the disclosure in detail, it is to be understood that the invention is not limited in its application to the details set forth in the following description or exemplified by the Examples. The invention is capable of other embodiments or of being practiced or carried out in various ways. Also, it is to be understood that the phraseology and terminology employed herein is for the purpose of description and should not be regarded as limiting.

### Overview of exemplary system

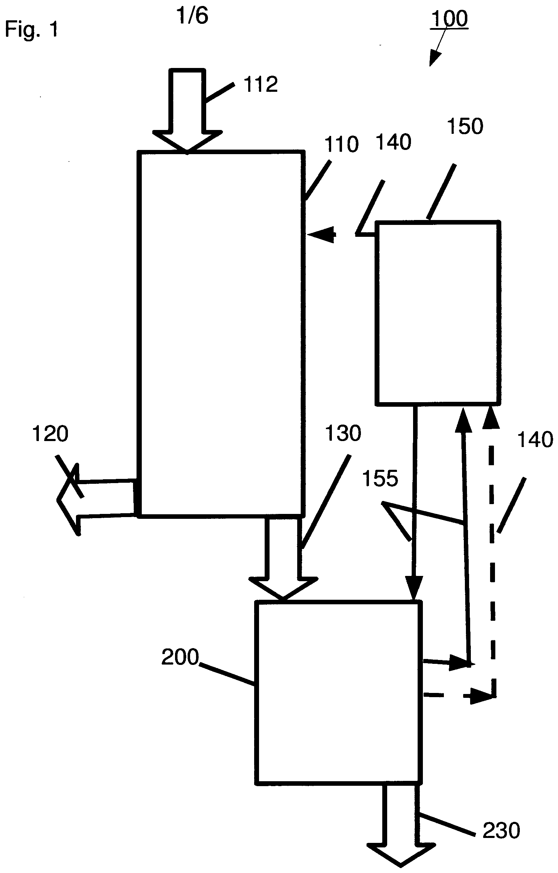

Fig. 1 is a simplified schematic diagram of a system for acid hydrolysis of a lignocellulosic substrate indicated generally as 100. Depicted system 100 includes a main hydrolysis reactor 110 adapted to receive a lignocellulosic substrate input 112. Optionally, substrate 112 is provided as wood chips, although any "woody material" as described in the background can be used instead of wood.

Substrate 112 is brought into contact with a concentrated HCl solution in reactor 110 and hemicellulose and/or cellulose in the substrate are hydrolyzed to produce a mixture of soluble sugars and residual lignin. These materials are collected separately as lignin stream 120 and sugar mixture 130, each of which contains a large amount of HCl.

Since the acid acts as a catalyst, it is not consumed in the process. In addition, residual acid content of the product and the co-products should be low in order to enable their use. Acid recovery from the hydrolyzate should be conducted under conditions minimizing thermal degradation.

Details of exemplary hydrolysis methods and systems are described in detail in co-pending US provisional applications 61/483,777 and 61/487,319 (both publicly available, for instance, via the online file wrapper for WO2012/137204)

This application is primarily concerned with processing of sugar mixture 130. The processing includes removal of HCl and/or adjustment of the mixture to achieve one or more desired ratios of mixture components (e.g. disaccharides and/or monosaccharides). This processing is conducted in a sugar refining module, designated here generically as 200.

Optionally, additional sugar mixture is recovered from lignin stream 120 as described in co-pending US provisional application US 61/491,243 (publicly available, for instance, via the online file wrapper for WO2012/137204). In some exemplary aspects of the disclosure, this additional sugar mixture is routed to refining module 200. According to various exemplary aspects of the disclosure this additional sugar mixture increase a total sugar yield and/or changes a composition of the mixture.

As will be explained in greater detail hereinbelow, refining module 200 employs a flow of organic solvent 155 (solid arrows) to extract HCl 140 (dashed arrows) from sugar mixture 130.

De-acidified sugars 230 are the primary product of refining module 200. Module 200 also produces a stream of HCl 140 mixed with solvent 155 (depicted as parallel dashed and solid arrows respectively for clarity) which is routed to a solvent/HCl recovery module 150. Recovery module 150 separates HCl 140 from solvent 155. In some exemplary aspects of the disclosure, separation is by distillation. HCl 140 is recycled to hydrolysis reactor 110 and solvent 155 is recycled to refining module 200.

De-acidified sugars 230 can be used in various industrial conversion processes as described hereinbelow. Optionally, additional adjustments are made on de-acidified sugar 230 prior to these conversion processes as described below.

### Exemplary sugar mixtures:

Some exemplary aspects of the disclosure relate to sugar mixtures. In some exemplary aspects of the disclosure, the mixtures can be characterized as: containing monosaccharides (e.g. hexoses such as glucose and/or galactose and/or mannose) and having an oligosaccharides to total saccharides ratio ≥ 0.06. Optionally, the mixture includes disaccharides and a disaccharides to total saccharides ratio ≥ 0.05. Optionally, the mixture includes a pentose (e.g. xylose and/or arabinose and/or ribose and/or lyxose) and a pentose to total saccharides ratio ≥ 0.05. In some exemplary aspects of the disclosure, the mixture includes at least one alpha-bonded di-glucose (glucose bonded to glucose) and/or at least one beta-bonded di-glucose. Optionally, the mixture is characterized by a higher oligosaccharides to total saccharide ratio ≤ 0.2.

According to various exemplary aspects of the disclosure the alpha-bonded di-glucose includes one or more of maltose, isomaltose and trehalose and the beta-bonded di-glucose includes one or more of gentiobiose, sophorose and cellobiose.

In some exemplary aspects a ratio of at least one of said alpha-bonded di-glucose and said beta-bonded di-glucose relative to the total saccharide content is ≥ 0.01, optionally ≥ 0.03.

In some exemplary aspects, the alpha-bonded di-glucose includes maltose and/or isomaltose and/or trehalose and/or kojibiose and/or nigerose. In some exemplary aspects, the beta-bonded di-glucose includes gentiobiose and/or sophorose and/or cellobiose and/or laminaribiose and/or beta-trehalose.

In some exemplary aspects, a proportion of the alpha-bonded di-glucose and/or the beta-bonded di-glucose is at least 0.01, optionally at least 0.02, optionally at least 0.03 relative to total saccharides.

According to some exemplary aspects, the mixture includes multiple alpha-bonded di-glucoses and the combined proportion of these di-glucoses to total saccharides is at least 0.03. According to still another aspects, the mixture includes multiple beta-bonded di-glucoses and the combined proportion to total saccharides of these beta-bonded di-glucoses is at least 0.03.

### Exemplary methods to make such mixtures:

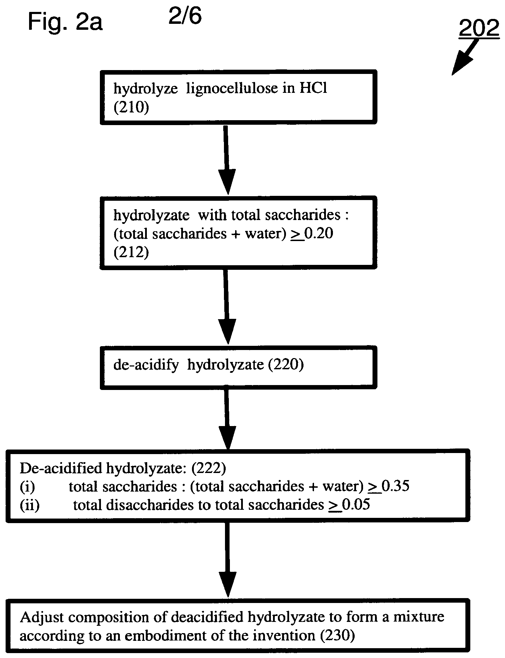

Fig. 2a is a simplified flow diagram depicting an exemplary method of making sugar mixtures as described above indicated generally as 202. According to the depicted method a lignocellulosic material is hydrolyzed 210 in HCl. In some exemplary aspects of the disclosure, and in the method of the invention, hydrolysis 210 employs a medium containing a ratio of HCl to (HCl + water) ≥ 0.37 by weight. Hydrolysis 210 yields a hydrolyzate 212 comprising total saccharides to (total saccharides + water) ≥0.20 by weight. The depicted method also includes de-acidifying 220 hydrolyzate 212 to form a de-acidified hydrolyzate 222 comprising: (i) a ratio of total saccharides to (total saccharides + water) ≥0.35 and; (ii) a ratio of total disaccharides to total saccharides ≥0.05. Optionally, an increase in saccharide concentration resulting from de-acidification 220 contributes to an increase in value.

Method 202 also includes adjusting 230 a composition of de-acidified hydrolyzate 222 to form a sugar mixture as described above. According to various exemplary aspects of the disclosure adjusting 230 includes, but is not limited to, one or more of concentration, dilution, polishing, active carbon treatment, ion exchange chromatography and enzymatic oligomerization (e.g. to form dimers). Optionally, glycosyltransferases are employed.

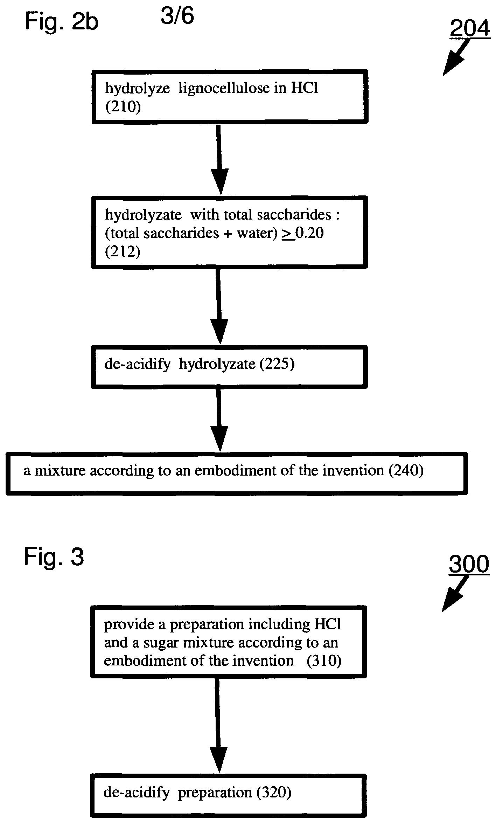

Fig. 2b is a simplified flow diagram depicting an additional exemplary method of making sugar mixtures as described above indicated generally as 204. Depicted method 204 includes hydrolyzing 210 to produce hydrolyzate 212 as described for method 202.

However, in depicted method 204 de-acidifying 225 of hydrolyzate 212 yields a de-acidified hydrolyzate comprising a mixture 240 according to an exemplary aspect of the disclosure as described above.

With regards to methods 202 and/or 204 hydrolysis 210 is optionally conducted in a counter-current mode of operation and/or at a temperature of less than 25°C. In some exemplary aspects of the disclosure, the lignocellulosic material hydrolyzed includes softwood, optionally pine.

According to various exemplary aspects of methods 202 and/or 204 de-acidifying 220 or 225 includes selective extraction of HCl with an alcohol. In some exemplary aspects of the disclosure, the alcohol has a water solubility of less than 15%, e.g. an alcohol with 5 to 8 carbon atoms. Optionally, some water is extracted with the HCl. Optionally; the de-acidifying is conducted at a temperature of less than 80°C.

### Exemplary de-acidification methods

Fig. 3 is a simplified flow diagram depicting an exemplary method of de-acidifying a sugar mixture as described above indicated generally as 300. According to the depicted method a preparation including HCl and a sugar mixture as described above is provided 310 and de-acidified 320. In some exemplary aspects of the disclosure, de-acidifying 320 includes selective extraction of HCl with an alcohol. Optionally, hexanol or 2-ethyl-1-hexanol is employed for this extraction. Optionally, de-acidifying 320 is conducted at a temperature of less than 80°C, optionally less than 70°C and optionally less than 60°C.

As used herein, "selective extraction" indicates that the HCl/carbohydrate ratio in the extract is greater than that ratio in the acidic hydrolyzate, optionally at least 5 fold greater, optionally at least 10 fold.

In some exemplary aspects of the disclosure, de-acidifying is conducted according to methods disclosed in co-pending Israeli patent application IL 206,152.

### Exemplary downstream processing of sugar mixtures

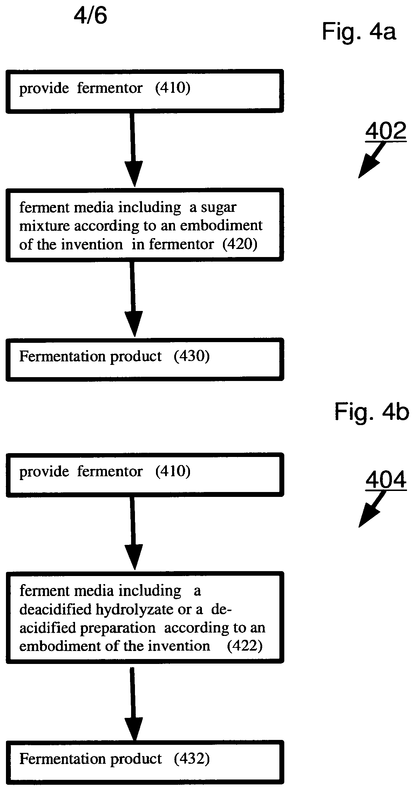

Fig. 4a is a simplified flow diagram depicting an exemplary method of producing a fermentation product from a sugar mixture as described above indicated generally as 402. According to the depicted method a fermentor is provided 410 and a media comprising a sugar mixture as described above is fermented 420 to produce a fermentation product 430.

Fig. 4b is a simplified flow diagram depicting an exemplary method of producing a fermentation product from a sugar mixture as described above indicated generally as 404. According to the depicted method a fermentor is provided 410 and a media comprising a de-acidified hydrolyzate as described above or a de-acidified preparation as described above is fermented 422 to produce a fermentation product 432.

According to various exemplary aspects of the disclosure fermentation product 430 and/or 432 includes an alcohol and/or a carboxylic acid and/or an amino acid and/or a monomer for the polymer industry and/or a protein.

One common fermentation product is ethanol, which may be useful, for example, as a fuel. For example, ethanol may be added to gasoline to produce "gasohol" as is commonly done in the United States, or used as a fuel itself as commonly done in Brazil.

Optionally, the fermentation product is a protein. In some exemplary aspects of the disclosure, the protein is a heterologous protein and a disaccharide in the sugar mixture triggers an inducible regulatory element in a construct containing a sequence encoding the protein. In some exemplary aspects, increasing an amount and/or ratio of a specific disaccharide in the sugar mixture contributes to an increased yield of fermentation product 430 and/or 432 per unit of sugar mixture in the media fermented 420 and/or 422.

### Additional methods including enzymatic hydrolysis

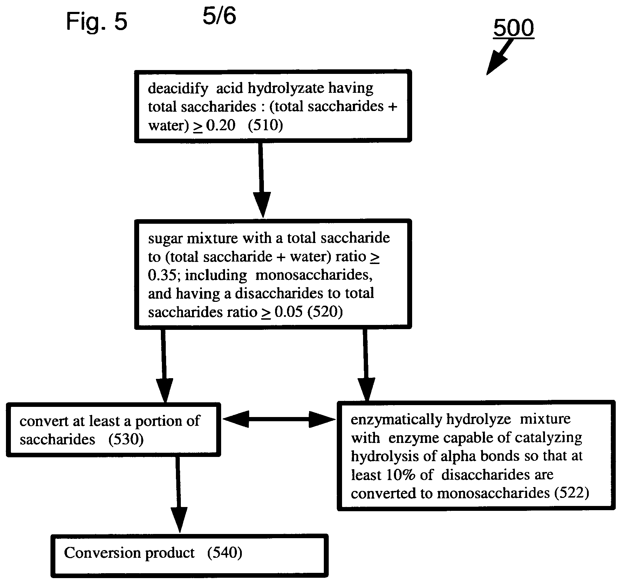

Fig. 5 is a simplified flow diagram depicting an exemplary method for the production of a conversion product from a sugar mixture as described above indicated generally as 500. According to the depicted method, an acid hydrolyzate (as described above) including total saccharides to (total saccharides + water) ≥ 0.20 by weight is de-acidified 510 to produce a sugar mixture 520 with a total saccharide to (total saccharide + water) ratio ≥ 0.35 including monosaccharides and having a disaccharides to total saccharides ratio ≥ 0.05. This mixture is an exemplary sugar mixture as described above.

Depicted exemplary method 500 also includes enzymatically hydrolyzing 522 a sugar mixture with an enzyme capable of catalyzing hydrolysis of alpha bonds in the mixture so that at least 10%, optionally at least 20%, optionally at least 30% of the disaccharides are converted to monosaccharides. The phrase "capable of catalyzing hydrolysis of alpha bonds" indicates any enzyme with at least 5% of the activity of alpha amylase with respect to alpha bonds. Optionally, at least 10% of the di-saccharides and higher saccharides are hydrolyzed to release additional monosaccharides. Optionally, higher saccharides release additional disaccharides.

In some exemplary aspects of the disclosure, enzymatically hydrolyzing 522 is performed without having to concentrate dilute saccharide solutions, i.e. the enzymatic hydrolysis is performed at relatively high sugar concentration.

The depicted method also includes converting 530 at least a portion of the saccharides to a conversion product 540.

According to various exemplary aspects of the disclosure converting includes fermentation and/or chemical conversion. Chemical conversion can be, for example, catalytic conversion.

According to various exemplary aspects of the disclosure, converting and/or enzymatic hydrolysis may each be conducted in multiple stages in various sequences. Optionally, one or more converting processes are conducted between enzymatic hydrolysis stages. Alternatively or additionally, one or more enzymatic hydrolysis stages are conducted between converting processes.

In some exemplary aspects of the disclosure, the sugar mixture comprises oligosaccharides with at least three sugar units.

In some exemplary aspects of the disclosure, the initial acid hydrolyzate results from counter-current hydrolysis. Optionally, the acid hydrolysis is conducted at a temperature of less than 25°C.

In some exemplary aspects of the disclosure, de-acidifying 510 includes extraction with an extractant including an alcohol. Optionally, de-acidifying 510 is conducted at a temperature of less than 80°C.

In some exemplary aspects of the disclosure, enzymatically hydrolyzing 522 includes hydrolyzing beta bonds.

According to various exemplary aspects of the disclosure enzymatically hydrolyzing 522 includes use of at least one of one enzyme belonging to EC 3.2.1 according to the Nomenclature Committee of the International Union of Biochemistry and Molecular Biology (NC-IUBMB) Enzyme Nomenclature Recommendations. This class of enzymes includes, but is not limited to, amylases cellulases, hemicellulases, transglucosidases, glucoamylases, alpha-glucosidases and pullulanases. Optionally, an alpha amylase and/or a beta amylase and/or a 1-4 alpha glucosidase are employed.

Optionally, one or more of these enzymes may be provided as part of an enzyme cocktail and/or a cellular extract including other undefined enzymes.

In some exemplary aspects of the disclosure, an immobilized enzyme is used for enzymatically hydrolyzing 522. Optionally, immobilization contributes to hydrolysis of a greater number of bonds per enzyme molecule.

In some exemplary aspects of the disclosure, at least a portion of converting 530 is conducted simultaneously with enzymatically hydrolyzing 522. Optionally, this concurrent processing prevents buildup of hydrolysis products which might adversely affect enzyme hydrolysis kinetics. For purposes of this specification and the accompanying claims the term "simultaneously" is used in its art accepted sense (i.e. simultaneous saccharification and fermentation).

Optionally, a ratio of total saccharides to (total saccharides + water) ≥ 0.15, optionally ≥ 0.2 is maintained during enzymatic hydrolyzation.

According to some exemplary aspects, enzymatically hydrolyzing 522 includes a single reaction, or temporally and/or distinct reactions, which reactions differ, in the composition of enzymes employed and/or in temperature and/or in pH.

In some exemplary aspects of the disclosure, enzymatically hydrolyzing 522 includes fermentation with micro-organisms that produce the desired enzyme(s).

Alternatively or additionally, converting 530 comprises fermentation.

Optionally, sugar mixture 520 includes at least one pentose and a ratio between the at least one pentose and total saccharides is ≥ 0.05 by weight.

### Exemplary de-acidification processes

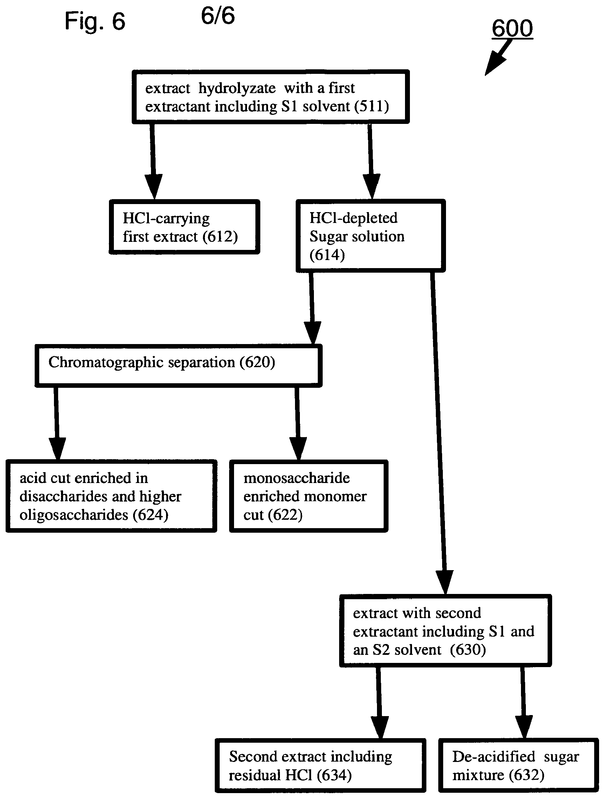

Fig. 6 is a simplified flow diagram which illustrates some exemplary ways in which de-acidification 510 of Fig. 5 can be accomplished.

In the depicted exemplary aspects of the disclosure, and in the specific method of the invention, de-acidifying 510 includes extracting 511 the hydrolyzate with a first extractant including an S1 solvent to form an HCl-carrying first extract 612 and an HCl-depleted sugar solution 614. Optionally, the first extractant includes 70, 80, 90, 95 or substantially 100% of the S1 solvent. However, HCl may be present in sugar solution 614 at an unacceptable level. If that is the case, one or more additional separation strategies can optionally be employed.

According to various exemplary aspects of the disclosure, and specifically in the method of the invention, residual HCl in HCl-depleted sugar solution 614 is removed by chromatographic separation 620 and/or by a subsequent extraction 630 with a second extractant including S1 and a second solvent (S2). Optionally, the total solvent concentration in the second extractant is 50, 60, 70, 80, 90, 95 or substantially 100% or intermediate percentages.

In those exemplary aspects of the disclosure which employ chromatographic separation 620 sugar solution 614 is separated into an HCl containing "acid cut" 624 which is enriched in disaccharides and higher saccharides relative to total saccharides and a "monomer cut" 622 enriched in monosaccharides relative to total saccharides. In some exemplary aspects of the disclosure, acid cut 624 is subject to further treatment to separate saccharides from HCl and/or to adjust a ration of monosaccharides to total saccharides. Exemplary chromatographic separation techniques are disclosed in co-pending application IL 211093.

In those exemplary aspects of the disclosure which employ a subsequent extraction 630, there is a selective transfer of HCl to the second extractant to form a second extract 634 and the de-acidified hydrolyzate 520 depicted here as de-acidified sugar mixture 632. Exemplary subsequent extraction techniques are disclosed in co-pending PCT application IL2011/000130.

Optionally, the S1 solvent employed in the initial extracting 511 and the subsequently extracting 630 includes a same solvent and/or a different solvent. S1 solvents employed in exemplary aspects of the disclosure include, but are not limited to alcohols (e.g. hexanol and 2-ethyl hexanol), ketones and aldehydes having at least 5 carbon atoms and combinations thereof. In the method of the present invention, the S1 solvent is a solvent selected from the group consisting of alcohols, ketones and aldehydes having at least 5 carbon atoms.

In some exemplary aspects of the disclosure, the second extractant is characterized by a delta-P greater than the delta-P of the first extractant by at least 0.2 MPa^{1/2} and/or a delta-H greater than the delta-H of the first extractant by at least 0.2 MPa^{1/2}.

According to various exemplary aspects of the disclosure, S2 includes at least one member selected from the group consisting of C1-C4 mono- or poly-alcohols, aldehydes and ketones. In the method of the present invention, the S2 solvent is selected from the group consisting of C1-C4 mono- or poly-alcohols, aldehydes and ketones

In some exemplary aspects of the disclosure, a ratio of HCl to total saccharides in the de-acidified hydrolyzate 520 and/or 632 is ≤ 0.03 by weight.

### Exemplary industrial contexts of downstream processing

Potential downstream applications of soluble carbohydrates include, but are not limited to, production of bio-fuels (e.g. ethanol, butanol or hydrocarbons), use in the food industry (e.g. fermentation to citric acid or xanthan gum and conversion of xylose to xylitol for use as an artificial sweetener) and industrially useful monomers.

As new processes are developed for the production of alternative fuels such as fatty acid esters and hydrocarbons (directly formed by fermentation or produced by conversion of fermentation products), the demand for soluble carbohydrates is expected to increase.

By way of example, sugar mixtures according to various exemplary aspects of the disclosure are expected to be useful in fermentors which employ inducible promoters such as that described in US 7,713,725 for example.

Alternatively or additionally, sugar mixtures according to various exemplary aspects of the disclosure are expected to be useful in production of fatty ester compositions such as that described in, for example, US 2010/0071259.

Alternatively or additionally, sugar mixtures according to various exemplary aspects of the disclosure are expected to be useful in extractive fermentation such as described in, for example, WO 2009/042950.

Alternatively or additionally, sugar mixtures according to various exemplary aspects of the disclosure are expected to be useful in production of fatty acid derivatives as described in, for example, WO 2008/119082.

Alternatively or additionally, sugar mixtures according to various exemplary aspects of the disclosure are expected to be useful in production of peptides as described in, for example, US 7595173.

In summary, direct downstream products of sugar mixtures according to various exemplary aspects of the disclosure resulting from fermentation and/or conversion (e.g. alcohols, lactic acid, acrylic acid and antimicrobial peptide) are expected to give rise to a wide variety of products resulting from further processing of these conversion products. Such products include, but are not limited to, automobile fuel (e.g. for automobiles and/or airplanes), diapers, plastic consumer products and paint.

### Exemplary consumer products

Some exemplary aspects of the disclosure, relate to consumer products and their manufacture or preparation.

In some exemplary aspects of the disclosure, a sugar mixture according to one exemplary aspect of the disclosure is converted to a fermentation product according to one or more additional aspects of the disclosure. Optionally, the fermentation product includes at least one member selected from the group consisting of alcohols, carboxylic acids, amino acids, monomers for the polymer industry and proteins.

In some exemplary aspects, the fermentation product is processed to produce a consumer product. Exemplary consumer products include but are not limited to detergent, polyethylene-based products, polypropylene-based products, polyolefin-based products, polylactic acid (polylactide)-based products, polyhydroxyalkanoate-based products and polyacrylic-based products.

In some exemplary aspects, the detergent includes a sugar-based surfactant and/or a fatty acid-based surfactant and/or a fatty alcohol-based surfactant and/or a cell-culture derived enzyme.

In some exemplary aspects, the polyacrylic-based product includes a plastic and/or a floor polish and/or a carpet and/or a paint and/or a coating and/or an adhesive and/or a dispersion and/or a flocculants and/or an elastomer and/or acrylic glass and/or an absorbent articles (e.g. incontinence pads, sanitary napkins, feminine hygene products, and diapers).

Polyolefin-based products may be, for example, milk jugs, detergent bottles, margarine tubs, garbage containers, water pipes, absorbent articles, diapers, non woven fabrics (e.g. pre-moistened towellettes), HDPE toys and HDPE detergent packagings.

In some exemplary aspects, a polypropylene based product is provided as an absorbent articles, optionally a diaper.

In other exemplary aspects, a polypropylene based product is provided as anon woven fabric item, optionally a pre-moistened towellette.

Polylactic acid based products may be, for example, packaging of agriculture or dairy products, plastic bottles, biodegradable products and disposables.

Polyhydroxyalkanoate based products may be, for example, packaging of agriculture products, plastic bottles, coated papers, molded or extruded articles, feminine hygiene products, tampon applicators, absorbent articles, disposable nonwovens and wipes, medical surgical garments, adhesives, elastometers, films, coatings, aqueous dispersants, fibers, intermediates of pharmaceuticals and binders.

Optionally, the fermentation product includes at least one member of the group consisting of ethanol, butanol, isobutanol, a fatty acid, a fatty acid ester, a fatty alcohol and biodiesel. In some exemplary aspects of the disclosure, the fermentation product is processed to produce an isobutene condensation product and/or a jet fuel and/or gasoline and/or gasohol and/or diesel fuel and/or drop-in fuel and/or a diesel fuel additive and/or a precursor thereof. According to various exemplary aspects of the disclosure gasohol is ethanol-enriched gasoline or butanol-enriched gasoline.

Optionally, the product is a diesel fuel. Optionally, the product is gasoline. Optionally, the product is jet fuel. Optionally, the product is a drop-in fuel.

According to various exemplary aspects of the disclosure a consumer product, a precursor of a consumer product, or an ingredient of a consumer product is produced from a fermentation product. Optionally, the consumer product, precursor of a consumer product, or ingredient of a consumer product includes at least one fermentation product as described hereinabove including one or more of carboxylic and fatty acids, dicarboxylic acids, hydroxylcarboxylic acids, hydroxyl di-carboxylic acids, hydroxyl-fatty acids, methylglyoxal, mono-, di-, or poly-alcohols, alkanes, alkenes, aromatics, aldehydes, ketones, esters, biopolymers, proteins, peptides, amino acids, vitamins, antibiotics, and pharmaceuticals. In some exemplary aspects of the disclosure, the product is ethanol-enriched gasoline, jet fuel, or biodiesel.

In some exemplary aspects of the disclosure, a consumer product, or an ingredient of a consumer product according as described above has a ratio of carbon-14 to carbon-12 of 1.8 x 10⁻¹³, optionally 2.0 x 10⁻¹³ or greater.

In some exemplary aspects of the disclosure, the consumer product includes an ingredient as described above and an additional ingredient produced from a raw material other than lignocellulosic material. Optionally, the ingredient and the additional ingredient are essentially of the same chemical composition.

Some exemplary aspects of the disclosure relate to a consumable product as described above including a marker molecule at a concentration of at least 100ppb.
The marker molecule optionally includes one or more of furfural, hydroxy-methyl furfural, products of furfural or hydroxy-methylfurfural condensation, color compounds derived from sugar caramelization, levulinic acid, acetic acid, methanol, galcturonic acid, and glycerol.

It is expected that during the life of this patent many EC 3.2.1 enzymes will be characterized and the scope of the invention is intended to include all such new enzymes *a priori.*

Specifically, a variety of numerical indicators have been utilized. It should be understood that these numerical indicators could vary even further based upon a variety of engineering principles, materials, intended use and designs incorporated into the invention. Additionally, components and/or actions ascribed to exemplary embodiments of the invention and depicted as a single unit may be divided into subunits. Conversely, components and/or actions ascribed to exemplary aspects of the disclosure and depicted as sub-units/individual actions may be combined into a single unit/action with the described/depicted function.

Alternatively, or additionally, features used to describe a method can be used to characterize an apparatus and features used to describe an apparatus can be used to characterize a method.

It should be further understood that the individual features described hereinabove can be combined in all possible combinations and sub-combinations to produce additional aspects of the disclosure. The examples given above are exemplary in nature and are not intended to limit the scope of the invention which is defined solely by the following claims. Specifically, the invention has been described in the context of ethanol production but is widely applicable to any fermentation or conversion process.

The terms "include", and "have" and their conjugates as used herein mean "including but not necessarily limited to".

### EXAMPLES

Reference is now made to the following examples, which together with the above descriptions, illustrate the invention in a non-limiting fashion.

### MATERIALS AND METHODS

The following materials and methods are used in performance of experiments described in examples hereinbelow:
Enzymes: Some enzymes (Examples 1-3) were from Novozymes (Novozymes A/S; Denmark): Spyrizyme Fuel HS: Glucoamylase with α 1-4 and 1-6 activity; 1425 AGU/g declared activity; Light to dark-brown liquid
Liquozyme SC DS: α-Amylase with 1-4 activity; 240 KNU-S/g declared activity; Amber liquid. Manufaturer's instructions are:
   Spyrizyme Fuel HS : 0.025-0.035% (w/w) grain "as is"
   Liquozyme SC DS : n.a.
   Cellic CTec : 1.5%, 3.0%, and 15% w/w (g enzyme/g cellulose).
   Cellic HTech : 0.1-0.5% (w/w TS).
Additional enzymes were obtained from Genencor (Danisco; Genencor, Beloit WI, USA): Spezyme Alpha: <1% α-Amylase activity 13,775 (Alpha Amylase Units)/g (min.), thermostable, dark-brown liquid, optimal pH 5.5-6.5.
Transglucosidase L-2000: 1-5% Active enzyme, having α 1-4 activity, yellow-brown liquid, optimal pH 4.5-5.5.
The recommended usages of enzymes (A. Kelley, Genencor) are:
   Spezyme Alpha: 0.025%
   Transglucosidase L-2000: 0.01%
   Accelerase BG: 8%
   Accelerase DUET: 8%

***Hydrolyzates:*** Hydrolyzates from Pinewood were employed as a substrate for enzymatic analyses. The hydrolyzates were adjusted to contain a desired % **TS (Total Suaccharides).** All experiments were conducted at pH 4.0-5.1 at various temperatures from 50 to 85°C. Loading doses of the enzymes were also varied.

In all of the enzymatic hydrolysis experiments time 0 (hrs.) refers to the blank (without enzyme).

Hydrolyzates from pinewood, sugarcane bagasse and eucalyptus wood were also subject to detailed analyses of monosaccharide and disaccharide concentrations.

*Control of reaction conditions:* The hydrolyzate was diluted with deionized (DI) water and buffer to a desired TS% and pH. The enzyme solution was added and the flask was kept at a selected temperature with stirring throughout incubation. Samples were withdrawn according to an experimental schedule and the enzyme was denaturated by heating and filtered out.

### Saccharide analyses:

Saccharride composition of the samples was analyzed by HPLC using a Varian Prostar® and a Rezex RSO-Oligosaccharide Ag+, 10 x 200mm column and pre-column in the following conditions:
Column Temp.: 80°C, Mobile Phase: Water (HPLC grade), Flow Rate: 0.3mL/min, Injection: 5 - 10µL (depending on sugars conc.), Detector: RI, Detector Temp.: 40°C. The DP groups HPLC results are given in area%, the x-axis in the graphs represents time (hrs.) and the y-axis area%.

DP stands for degree of polymerization, so that DP1 refers to all mono-saccharides, DP2 are all di-saccharides, DP3 are all tri-saccharides and DP>3 refers to oligosaccharides containing more than three sugar units (DP groups given as %area).

### Example 1:

### Exemplary Enzymatic Hydrolysis of a De-acidified Hydrolyzate with 30% TS using Spirizyme

In order to examine the influence of enzyme amounts on total monosaccharide yield, enzyme amount was titrated and efficiency of enzymatic hydrolysis was measured.

In a first series of enzymatic hydrolysis experiment Spyrizyme Fuel HS and Liquozyme SC DS were employed.

A pinewood hydrolyzate with 30% TS at pH 4.9 was hydrolyzed with 3mg enzyme solution/lgr 100% sugar at a temperature of 62°C with stirring (enzyme was 0.3%). Results are summarized in Table 1.

**Table 1: Spirizyme Fuel HS (3mg enzyme solution/lgr 100% sugar)**

| Time (Hr) | DP1 (%area) | DP2 (%area) | DP3 (%area) | DP>3 (%area) |
|---|---|---|---|---|
| 0 | 45.7 | 31.8 | 13.7 | 8.8 |
| 2.5 | 51.2 | 31.5 | 12.2 | 5.0 |
| 4.0 | 51.4 | 31.5 | 11.8 | 5.3 |
| 24 | 56.2 | 28.2 | 11.0 | 4.6 |

In order to assess the impact of increasing the amount of enzyme, 33mg enzyme solution/lgr 100% sugar were employed. Results are summarized in Table 2.

**Table 2: Spirizyme Fuel HS (33mg enzyme solution/lgr 100% sugar)**

| Time (hr) | DP1 (%area) | DP2 (%area) | DP3 (%area) | DP>3 (%area) |
|---|---|---|---|---|
| 0 | 45.7 | 31.8 | 13.7 | 8.8 |
| 2.5 | 64.0 | 26.8 | 7.0 | 2.3 |
| 4.0 | 65.1 | 26.2 | 6.4 | 2.3 |

Results summarized in Tables 1 and 2 demonstrate a trend towards increased proportion of DP1 and DP2 saccharides at the expense of DP3 and DP>3 saccharides. This trend increased as a function of time.

In order to assess the impact of increasing the amount of enzyme even further, 65.6 mg enzyme solution/lgr 100% sugar were employed. Results are summarized in Table 3.

**Table 3: Spirizyme Fuel HS (65.6mg enzyme solution/lgr 100% sugar)**

| Time (hr) | DP1 (%area) | DP2 (%area) | DP3 (%area) | DP>3 (%area) |
|---|---|---|---|---|
| 0 | 45.7 | 31.8 | 13.7 | 8.8 |
| 2.5 | 68.2 | 26.8 | 7.0 | 2.3 |
| 4.0 | 68.1 | 26.2 | 6.4 | 2.3 |

Results presented in Table 3 indicate that it is possible to increase the yield of monosaccharides even further.

In order to assess the impact of increasing the amount of enzyme even further, 282.6 mg enzyme solution/lgr 100% sugar were employed. Results are summarized in Table 4.

**Table 4: Spirizyme Fuel HS (282.6mg enzyme solution/lgr 100% sugar)**

| Time (hr) | DP1 (%area) | DP2 (%area) | DP3 (%area) | DP>3 (%area) |
|---|---|---|---|---|
| 0 | 45.7 | 31.8 | 13.7 | 8.8 |
| 2.5 | 70.6 | 22.4 | 5.6 | 1.5 |
| 4.0 | 71.1 | 21.6 | 5.5 | 1.8 |

Results summarized in Tables 1 to 4 indicate that enzymes capable of hydrolyzing alpha bonds can be employed to increase monosaccharide concentrations in acid hydrolyzates of cellulose. The enzyme dose of 282.6 mg gave the highest yield of mono-saccharides.

However, other conditions may contribute to enzymatic hydrolysis results. Exemplary other conditions include, but are not limited to, reaction temperature, exact nature of the sugars in the hydrolyzate in terms of both bond type and oligomer length distribution.

### EXAMPLE 2:

### Influence of %TS on Enzymatic Hydrolysis of a De-acidified Hydrolyzate using Spirizyme

In order to examine the influence of %TS in the substrate on total monosaccharide yield, enzyme amount was titrated again against a hydrolyzate similar to that used in Example 1, but with decreasing amounts of %TS and efficiency of enzymatic hydrolysis was measured.

A pinewood hydrolyzate with 15% TS at pH 5.1 was hydrolyzed with 72.8 mg enzyme solution/lgr 100% sugar at a temperature of 60°C with stirring (enzyme -243 of manufacturer's recommendation). Results are summarized in Table 5. This experiment confirms results presented in Table 2 of Example 1 and suggests that more dilute sugar solutions are more amenable to enzymatic hydrolysis.

**Table 5: Spirizyme Fuel HS (72.8 mg enzyme solution/lgr 100% sugar)**

| Time (hr) | DP1 (%area) | DP2 (%area) | DP3 (%area) | DP>3 (%area) |
|---|---|---|---|---|
| 0 | 45.7 | 31.8 | 13.7 | 8.8 |
| 2.5 | 71.3 | 20.6 | 6.1 | 2.1 |
| 4.0 | 71.8 | 21.0 | 4.9 | 2.3 |
| 5.8 | 71.8 | 20.2 | 5.7 | 2.3 |
| 24 | 71.1 | 24.3 | 3.4 | 1.2 |
| 50 | 76.0 | 17.9 | 4.7 | 1.4 |

A pinewood hydrolyzate with 5% TS at pH 5.0 was hydrolyzed with 67 mg enzyme solution/lgr 100% sugar at a temperature of 62°C with stirring (enzyme -233 times manufacturer's recommendation). Results are summarized in Table 6. This experiment confirms results presented in Table 2 of Example 1 and suggests that more dilute sugar solutions are more amenable to enzymatic hydrolysis.

**Table 6: Spirizyme Fuel HS (67 mg enzyme solution/lgr 100% sugar)**

| Time (hr) | DP1 (%area) | DP2 (%area) | DP3 (%area) | DP>3 (%area) |
|---|---|---|---|---|
| 0 | 49.8 | 31.3 | 12.9 | 6.1 |
| 2.5 | 72.0 | 20.4 | 5.4 | 2.2 |
| 24 | 75.4 | 17.8 | 5.0 | 1.8 |
| 50 | 74.7 | 19.7 | 4.3 | 1.3 |
| 72 | 77.8 | 16.2 | 4.8 | 1.2 |
| 96 | 77.1 | 15.7 | 4.9 | 2.3 |

Results presented in table 6 confirm that enzymes capable of hydrolyzing alpha bonds can be employed to increase monosaccharide concentrations in acid hydrolyzates of cellulose.

Comparison of results presented in table 6 with those presented in Tables 2 and 5 suggests that dilution of sugar solutions prior to enzymatic hydrolysis contributes to an increase in monosaccharide yield.

Again, the exact nature of the sugars in the hydrolyzate in terms of both bond type and oligomer length distribution may influence the total yield of monosaccharides.

### EXAMPLE 3:

### Enzymatic Hydrolysis of a De-acidified Hydrolyzate with 30% TS using Liquozyme SC DS

In order to determine whether the results presented above were enzyme specific an additional experiment was conducted on a 30% TS hydrolyzate using Liquozyme. A pinewood hydrolyzate with 30% TS at pH 6.0 was hydrolyzed with 282.6 mg enzyme solution/lgr 100% sugar at a temperature of 85°C with stirring (enzyme -942 times manufacturer's recommendation for Spirizyme). Results are summarized in Table 7. This experiment confirms results presented in Table 2 of Example 1 and suggests that more dilute sugar solutions are more amenable to enzymatic hydrolysis.

**Table 7: Liquozyme SC DS (282.6 mg enzyme solution/lgr 100% sugar)**

| Time (hr) | DP1 (%area) | DP2 (%area) | DP3 (%area) | DP>3 (%area) |
|---|---|---|---|---|
| 0 | 45.7 | 31.8 | 13.7 | 8.8 |
| 4.0 | 59.8 | 27.1 | 8.4 | 4.5 |

Results presented in table 7 indicate that the ability of enzymes capable of hydrolyzing alpha bonds to increase monosaccharide concentrations in acid hydrolyzates of cellulose is not specific to Spirizyme Fuel HS.

These results also suggest that Liquozyme SC DS provides a higher yield of mono-saccharides than Spirizyme Fuel HS at 282.6mg enzyme solution/lgr 100% sugar (see Table 4 for comparison). However, the desirability of one enzyme over another may also be influence by other considerations including, but not limited to, availability, purity, bond specificity and price.

### EXAMPLE 4:

### Enzymatic Hydrolysis of a De-acidified Sugarcane Bagasse Hydrolyzate with Various Enzymes

In order to examine the possibility of applying enzymatic hydrolysis to hydrolyzates from non-wood substrates, a series of experiments was conducted on hydrolyzates prepared from sugar cane bagasse. The hydrolyzates used contained different TS (5%, 20%), the experiments were conducted at pH 4.5-5.1, and at various temperatures (50°C and 60°C). The loading doses of the enzymes were also varied.

A first experiment was conducted using Spezyme Alpha at a concentration of_34.2mg enzyme solution/lgr 100% sugar, at a temperature of 60°C and at pH 5.1, applied to a bagasse hydrolyzate with 20% TS. Results are summarized in Table 8.

**Table 8: Spezyme Alpha applied to Baggase hydrolyzate TS 20% (34.2mg enzyme solution; about 137 times manufacturer's recommendation)**

| Time (hr) | DP1 (%area) | DP2 (%area) | DP3 (%area) | DP>3 (%area) |
|---|---|---|---|---|
| 0 | 52.0 | 35.4 | 10.6 | 1.9 |
| 2.5 | 52.0 | 34.9 | 10.8 | 2.3 |
| 4.0 | 53.6 | 34.2 | 10.1 | 2.1 |
| 24 | 52.4 | 35.1 | 10.3 | 2.2 |
| 50 | 51.7 | 35.1 | 10.9 | 2.3 |

An additional experiment was conducted using Transglucosidase L-2000 at a concentration of 14.8mg enzyme /lgr 100% sugar at a temperature of 60°C at pH 5.0 applied to a bagasse hydrolyzate with 20% TS. Results are summarized in Table 9.

**Table 9: Transglucosidase L-2000 applied to Baggase hydrolyzate TS 20% (14.8mg enzyme; about 148 times manufacturer's recommendation)**

| Time (hr) | DP1 (%area) | DP2 (%area) | DP3 (%area) | DP>3 (%area) |
|---|---|---|---|---|
| 0 | 52.0 | 35.4 | 10.6 | 1.9 |
| 2.5 | 57.9 | 29.7 | 10.0 | 2.4 |
| 4.0 | 62.0 | 26.8 | 9.1 | 2.2 |
| 24 | 62.7 | 27.0 | 8.5 | 1.8 |
| 50 | 62.2 | 27.1 | 8.7 | 2.0 |

Results summarized in Tables 8 and 9 indicate that Transglucosidase L-2000 provides an increase in mono-saccharides in the bagasse hydrolyzate while Spezyme Alpha does not provide such an increase.

The results from Transglucosidase L-2000 (Table 9) confirm that enzymes capable of hydrolyzing alpha bonds can be used to increase the yield of monosaccharides.

The negative results from Spezyme Alpha presented in table 8 suggest that there may be inhibitors present in the hydrolyzate. At this stage it is not clear whether these inhibitors are specific to the substrate subject to the initial acid hydrolysis (e.g. sugar cane bagasse as opposed to pine wood) or are specific to the enzyme.

### EXAMPLE 5:

### Saccharide Composition of Hydrolyzates of a First Pine Wood

In order to obtain a sugar mixture, dry first pinewood was introduced into a six stage hydrolysis reactor series in a counter-current operation as described in co-pending US provisional application 61/48377 filed May 9, 2011 and entitled "Hydrolysis systems and methods". This application is fully incorporated herein by reference.

Briefly, an aqueous solution of 42% HCl was introduced continually at a temperature of 10-15°C for 24 hours. The hydrolyzate was collected, HCl was removed by extraction and the deacidified hydrolyzate was concentrated to give a sugar composition. The composition was analyzed by HPLC, the sample's total sugars was 74.3%. Analysis results of monosaccharides and disaccharides are summarized in Tables 10 and 11 respectively. The results are calculated as % from sample's refractive total saccharides (%/RTS).

**Table 10: Monosaccharides in hydrolyzate of the first pine wood**

| Rhamnose | Arabinose | Galactose | Glucose | Xylose | Mannose | Sum |
|---|---|---|---|---|---|---|
| 0.1 | 1.6 | 2.7 | 27.7 | 7.0 | 7.4 | 46.5 |

**Table 11: Disaccharides in hydrolyzate of the first pine wood**

| Trehalose | Isomaltose | Gentiobiose | Cellobiose | Nigerose | Sophorose | Other DP2 | Sum |
|---|---|---|---|---|---|---|---|
| 1.4 | 4.8 | 1.0 | 0.7 | 0.4 | 0.2 | 14.5 | 22.9 |

Results summarized in table 10 illustrate the presence of pentoses such as Arabinose and Xylose and Rhamnose (de-oxy pentose). Since these pentoses are prone to degradation under harsh acidic conditions, their presence suggests that the counter current design of the hydrolysis reactor contributes to a "pentose sparing" effect.

Results summarized in Table 11 illustrate the presence of alpha-bonded di-glucose such as Trehalose and Isomaltose, as well as the presence of beta-bonded di-glucose such as Gentiobiose and Sophorose.

### EXAMPLE 6:

### Saccharide Composition of Hydrolyzates of a Second Pine Wood

Similarly, a second pine wood was hydrolyzed, deacidified and concentrated to 77% TS.

Analysis results of monosaccharides and disaccharides are presented in Tables 12 and 13 respectively. The results are calculated as % from sample's refractive total saccharides (%/RTS).

**Table 12: monosaccharides in hydrolyzate of the second wood**

| Arabinose | Galactose | Glucose | Xylose | Mannose | Sum |
|---|---|---|---|---|---|
| 0.3 | 0.8 | 36 | 8 | 1.0 | 46 |

**Table 13: results of disaccharides in hydrolyzate of the second wood**

| Trehalose | Isomaltose | Gentiobiose | Cellobiose | Nigerose | Maltose | Other DP2 | Sum |
|---|---|---|---|---|---|---|---|
| 1.1 | 6.6 | 2.1 | 2.1 | 0.4 | 0.4 | 28 | 41 |

The second pine wood also contained 16.7% higher oligosaccharides.

Results summarized in tables 12 and 13 confirm the presence of pentose, of alpha-bonded di-glucose and of beta-bonded di-glucose. There are differences in the saccharide profiles of hydrolyzates of the two pine sources.

### EXAMPLE 7:

### Saccharide Composition of Hydrolyzate Prepared from Non-Pine Wood Substrates

In order to examine the effect of substrate composition on hydrolyzate composition in terms of specific sugars, deacidified hydrolyzates prepared from sugar cane bagasse and Eucalyptus wood were analyzed as in Example 6 .

Analysis results of monosaccharides and disaccharides from the sugar cane bagasse hydrolyzate are presented in Tables 14 and 15 respectively. The results are calculated as % from sample's refractive total saccharides (%/RTS).

**Table 14: results of monosaccharides in hydrolyzate of sugar cane bagasse**

| Arabinose | Galactose | Glucose | Xylose | Mannose | Fructose | Sum |
|---|---|---|---|---|---|---|
| 2.2 | 7.2 | 48.7 | 4.9 | 4.8 | 2.4 | 70.2 |

**Table 15: results of disaccharides in hydrolyzate of sugar cane bagasse**

| Isomaltose | Gentiobiose | Maltose | Other DP2 | Sum |
|---|---|---|---|---|
| 4.5 | 0.6 | 2.9 | 27 | 35 |

Analysis results of monosaccharides and disaccharides from the eucalyptus wood hydrolyzate are presented in Tables 16 and 17 respectively. The results are calculated as % from sample's refractive total saccharides (%/RTS).

**Table 16: results of monosaccharides in hydrolyzate of Eucalyptus wood**

| Arabinose | Galactose | Glucose | Xylose | Mannose | Fructose | Sum |
|---|---|---|---|---|---|---|
| 2.6 | 7.24 | 46.1 | 8.27 | 5.83 | 3.38 | 73.42 |

**Table 17: results of disaccharides in hydrolyzate of sugar Eucalyptus wood**

| Isomaltose | Cellobiose | Maltose | Other DP2 | Sum |
|---|---|---|---|---|
| 7.1 | 1.85 | 2.16 | 11.11 | 22 |

Results presented in tables 14 to 17, taken together with results presented in tables 10 to 13 suggest that the substrate used for the initial acid hydrolysis can influence the saccharide profile of the resultant de-acidified hydrolyzate.

## Claims

1. A method comprising:
(a) hydrolyzing a lignocellulosic material in a medium containing HCl in a ratio to (HCl + water) ≥ 0.37 by weight to form a hydrolyzate comprising total saccharides in a ratio to (total saccharides + water) ≥ 0.20 by weight; and
(c) de-acidifying said hydrolyzate to form a de-acidified hydrolyzate comprising:
(i) monosaccharides, wherein the monosaccharides comprise a pentose in a ratio to total saccharides ≥ 0.05; and
(ii) oligosaccharides in a ratio to total saccharides ≥ 0.06, wherein the oligosaccharides comprise disaccharides in a ratio to total saccharides ≥ 0.05, wherein the disaccharides comprise at least one alpha-bonded di-glucose and at least one beta-bonded di-glucose;
wherein said de-acidifying comprises (i) extracting the hydrolysate with a first extractant including an S1 solvent to form an HCl-carrying first extract and an HCl-depleted sugar solution, and further comprises removing residual HCl in said HCl-depleted sugar solution by at least one of (ii-a) chromatographic separation and (ii-b) extraction with a second extractant including S1 and a second solvent S2;
wherein the S1 solvent is an organic solvent with a water solubility of less than 15% and **characterised by** a polarity related component of Hoy's cohesion parameter (delta-P) between 5 and 10 MPa^{1/2} and/or by a hydrogen bonding related component of Hoy's cohesion parameter (delta-H) between 5 and 20 MPa^{1/2}, and which is a solvent selected from the group consisting of alcohols, ketones and aldehydes having at least 5 carbon atoms; and
wherein the S2 solvent is an organic solvent with a water solubility of at least 30% and **characterised by** a delta-P greater than 8 MPa^{1/2} and/or a delta-H greater than 12 MPa^{1/2}, and which is a solvent selected from the group consisting of C1-C4 mono- or poly-alcohols, aldehydes and ketones.

2. The method according to claim 1, wherein the de-acidified hydrolyzate comprises total saccharides in a ratio to (total saccharides + water) ≥ 0.35.

3. The method according to claim 1 or 2, wherein the de-acidified hydrolyzate has a ratio of HCl to total saccharides of ≤ 0.03 by weight.

4. The method according to any one of the preceding claims, wherein the de-acidified hydrolyzate has higher oligosaccharides in a ratio to total saccharides ≤ 0.2.

5. The method according to any one of the preceding claims, wherein a ratio of at least one of said alpha- bonded di-glucose and said beta-bonded di-glucose relative to total saccharides is (i) > 0.01 in the de-acidified hydrolyzate or (ii) > 0.03 in the de-acidified hydrolyzate.

6. The method according to any one of the preceding claims, wherein said alpha-bonded di-glucose includes at least one of maltose, isomaltose and trehalose.

7. The method according to any one of the preceding claims, wherein said beta-bonded di-glucose includes at least one of gentiobiose, sophorose and cellobiose.

8. The method according to any one of the preceding claims, wherein the S1 solvent is hexanol or 2-ethyl hexanol.

9. The method according to any one of the preceding claims, which comprises removing residual HCl in the HCl-depleted sugar solution by (ii-a) chromatographic separation.

10. The method according to any one of the preceding claims, where said hydrolyzing step (a) forms said hydrolyzate and residual lignin, and said method further comprises: (b) separating said hydrolyzate from said residual lignin before said de-acidifying step (c).

11. The method according to any one of the preceding claims, which further comprises (d) enzymatically hydrolyzing the de-acidified hydrolyzate with an enzyme capable of catalyzing hydrolysis of alpha bonds in the mixture so that at least 10% of the disaccharides are converted to monosaccharides.

12. The method according to any one of the preceding claims, which further comprises
(e) providing a fermentor; and
(f) fermenting a medium comprising the de-acidified hydrolyzate in said fermentor to produce a fermentation product.

13. The method according to claim 12, wherein said method further comprises:
(g1) processing said fermentation product to produce a consumer product selected from the group consisting of detergent, polyethylene-based products, polypropylene-based products, polyolefin-based products, polylactic acid (polylactide)-based products, polyhydroxyalkanoate-based products and polyacrylic-based products; or
(g2) producing a consumer product comprising the fermentation product, wherein said fermentation product is at least one selected from carboxylic and fatty acids, dicarboxylic acids, hydroxylcarboxylic acids, hydroxyl di-carboxylic acids, hydroxyl-fatty acids, methylglyoxal, mono-, di-, or poly-alcohols, alkanes, alkenes, aromatics, aldehydes, ketones, esters, biopolymers, proteins, peptides, amino acids, vitamins, antibiotics, and pharmaceuticals; or
(g3) producing a consumer product comprising the fermentation product, wherein said consumer product is ethanol-enriched gasoline, jet fuel, or biodiesel.

## Patentansprüche

1. Verfahren, das Folgendes umfasst:
(a) Hydrolysieren eines Lignocellulosematerials in einem Medium, das HCl in einem Gewichtsverhältnis von (HCl + Wasser) ≤ 0,37 umfasst, unter Bildung eines Hydrolysats, umfassend Gesamtsaccharide in einem Gewichtsverhältnis von (Gesamtsacchariden + Wasser) ≤ 0,20 Gew.-%; und
(c) Entsäuern des Hydrolysats unter Bildung eines entsäuerten Hydrolysats, umfassend:
(i) Monosaccharide, wobei die Monosaccharide eine Pentose in einem Verhältnis zu Gesamtsacchariden von ≤ 0,05 umfassen; und
(ii) Oligosaccharide in einem Verhältnis zu Gesamtsacchariden von ≤ 0,06, wobei die Oligosaccharide Disaccharide in einem Verhältnis zu Gesamtsacchariden von ≤ 0,05 umfassen, wobei die Disaccharide mindestens eine alpha-gebundene Diglucose und mindestens eine beta-gebundene Diglucose umfassen;
wobei das Entsäuern Folgendes umfasst: (i) Extrahieren des Hydrolysats mit einem ersten Extraktionsmittel, das ein Sl-Lösungsmittel umfasst, um einen HCltragenden ersten Extrakt und eine HCl-abgereicherte Zuckerlösung zu bilden, und ferner das Entfernen von restlicher HCl in der HCl-abgereicherten Zuckerlösung durch (ii-a) chromatographische Trennung und/oder (ii-b) Extrahieren mit einem zweiten Extraktionsmittel, einschließlich S1 und einem zweiten Lösungsmittel S2;
wobei das SI-Lösungsmittel ein organisches Lösungsmittel mit einer Wasserlöslichkeit von weniger als 15% ist und **gekennzeichnet durch** eine polaritätsbezogene Komponente des Hoy-Kohäsionsparameters (Delta-P) zwischen 5 und 10 MPa^{1/2} und/oder durch eine wasserstoffbindungsbezogene Komponente des Hoy-Kohäsionsparameters (Delta-H) zwischen 5 und 20 MPa^{1/2} und die ein Lösungsmittel ist, ausgewählt aus der Gruppe bestehend aus Alkoholen, Ketonen und Aldehyden mit mindestens 5 Kohlenstoffatomen; und
wobei das S2-Lösungsmittel ein organisches Lösungsmittel mit einer Wasserlöslichkeit von mindestens 30% ist und
**gekennzeichnet durch** ein Delta-P größer als 8 MPa^{1/2} und/oder ein Delta-H größer als 12 MPa^{1/2} und das ein Lösungsmittel ist, ausgewählt aus der Gruppe bestehend aus C1-C4-Mono- oder Polyalkoholen, Aldehyden und Ketonen.

2. Verfahren nach Anspruch 1, wobei das entsäuerte Hydrolysat Gesamtsaccharide in einem Verhältnis zu (Gesamtsacchariden + Wasser) von ≤ 0,35 umfasst.

3. Verfahren nach Anspruch 1 oder 2, wobei das entsäuerte Hydrolysat ein Verhältnis von HCl zu Gesamtsacchariden von ≥ 0,03 Gew.-% aufweist.

4. Verfahren nach einem der vorhergehenden Ansprüche, wobei das entsäuerte Hydrolysat höhere Oligosaccharide in einem Verhältnis zu Gesamtsacchariden von ≥ 0,2 aufweist.

5. Verfahren nach einem der vorhergehenden Ansprüche, wobei ein Verhältnis der alphagebundenen Di-Glucose und/oder der beta-gebundenen Di-Glucose zu den Gesamtsacchariden (i) > 0,01 in dem entsäuerten Hydrolysat oder (ii) > 0,03 in dem entsäuerten Hydrolysat ist.

6. Verfahren nach einem der vorhergehenden Ansprüche, wobei die alpha-gebundene Di-Glucose Maltose, Isomaltose und/oder Trehalose umfasst.

7. Verfahren nach einem der vorhergehenden Ansprüche, wobei die Beta-gebundene Di-Glucose Gentiobiose, Sophorose und/oder Cellobiose umfasst.

8. Verfahren nach einem der vorhergehenden Ansprüche, wobei das S1-Lösungsmittel Hexanol oder 2-Ethylhexanol ist.

9. Verfahren nach einem der vorhergehenden Ansprüche, umfassend das Entfernen von restlicher HCl in der HCl-abgereicherten Zuckerlösung durch (ii-a) chromatographische Trennung.

10. Verfahren nach einem der vorhergehenden Ansprüche, wobei der Hydrolyseschritt
(a) das Hydrolysat und das restliche Lignin bildet und das Verfahren ferner Folgendes umfasst:
(b) Trennen des Hydrolysats vom restlichen Lignin vor dem Entsäuerungsschritt
(c).

11. Verfahren nach einem der vorhergehenden Ansprüche, das ferner Folgendes umfasst:
(d) enzymatisches Hydrolysieren des entsäuerten Hydrolysats mit einem Enzym, das die Hydrolyse von Alpha-Bindungen in der Mischung katalysieren kann, so dass mindestens 10 % der Disaccharide in Monosaccharide umgewandelt werden.

12. Verfahren nach einem der vorhergehenden Ansprüche, das ferner Folgendes umfasst:
(e) Bereitstellen eines Fermenters; und
(f) Fermentieren eines Mediums, das das entsäuerte Hydrolysat umfasst, im Fermenter, um ein Fermentationsprodukt herzustellen.

13. Verfahren nach Anspruch 12, wobei das Verfahren ferner Folgendes umfasst:
(g1) Verarbeiten des Fermentationsprodukts zur Herstellung eines Verbraucherprodukts, ausgewählt aus der Gruppe bestehend aus: Waschmittel, Produkte auf Polyethylenbasis, Produkte auf Polypropylenbasis, Produkte auf Polyolefinbasis, Produkte auf der Basis von Polymilchsäure (Polylactid), Produkte auf Polyhydroxyalkanoatbasis und Produkte auf Polyacrylbasis; oder
(g2) Herstellen eines Verbraucherprodukts, das das Fermentationsprodukt umfasst, wobei das Fermentationsprodukt ausgewählt wird aus Carbonsäuren und Fettsäuren, Dicarbonsäuren, Hydroxylcarbonsäuren, Hydroxyldicarbonsäuren, Hydroxylfettsäuren, Methylglyoxal-, Mono-, Di- oder Polyalkoholen, Alkanen, Alkenen, Aromaten, Aldehyden, Ketonen, Estern, Biopolymeren, Proteinen, Peptiden, Aminosäuren, Vitaminen, Antibiotika und/oder Pharmazeutika; oder
(g3) Herstellen eines Verbraucherprodukts, das das Fermentationsprodukt umfasst, wobei das Verbraucherprodukt mit Ethanol angereichertes Benzin, Düsentreibstoff oder Biodiesel ist.

## Revendications

1. Procédé, comprenant :
(a) l'hydrolyse d'un matériau lignocellulosique dans un milieu contenant HCl en un rapport à (HCl + eau) ≥ 0,37 en poids pour former un hydrolysat comprenant des saccharides totaux en un rapport à (saccharides totaux + eau) ≥ 0,20 en poids ; et
(c) la désacidification dudit hydrolysat pour former un hydrolysat désacidifié comprenant :
(i) des monosaccharides, dans lequel les monosaccharides comprennent un pentose en un rapport à saccharides totaux ≥ 0,05 ; et
(ii) des oligosaccharides en un rapport à saccharides totaux ≥ 0,06, dans lequel les oligosaccharides comprennent des disaccharides en un rapport à saccharides totaux ≥ 0,05, dans lequel les disaccharides comprennent au moins un di-glucose alpha-lié et au moins un di-glucose bêta-lié ;
dans lequel ladite désacidification comprend (i) l'extraction de l'hydrolysat avec un premier agent d'extraction incluant un solvant S1 pour former un premier extrait portant du HCl et une solution de sucre appauvrie en HCl, et comprend en outre l'enlèvement de HCl résiduel dans ladite solution de sucre appauvrie en HCl par au moins une de (ii-a) séparation chromatographique et (ii-b) extraction avec un second agent d'extraction incluant S1 et un second solvant S2 ;
dans lequel le solvant S1 est un solvant organique avec une hydrosolubilité inférieure à 15 % et **caractérisé par** un composant connexe à la polarité de paramètre de cohésion de Hoy (delta-P) entre 5 et 10 MPa^{1/2} et/ou par un composant connexe à la liaison d'hydrogène de paramètre de cohésion de Hoy (delta-H) entre 5 et 20 MPa^{1/2}, et qui est un solvant sélectionné parmi le groupe constitué de : alcools, cétones et aldéhydes ayant au moins 5 atomes de carbone ; et
dans lequel le solvant S2 est un solvant organique avec une hydrosolubilité d'au moins 30 % et **caractérisé par** un delta-P supérieur à 8 MPa^{1/2} et/ou un delta-H supérieur à 12 MPa^{1/2}, et qui est un solvant sélectionné parmi le groupe constitué de : C1-C4 mono- ou poly-alcools, aldéhydes et cétones.

2. Procédé selon la revendication 1, dans lequel l'hydrolysat désacidifié comprend des saccharides totaux en un rapport à (saccharides totaux + eau) ≥ 0,35.

3. Procédé selon la revendication 1 ou 2, dans lequel l'hydrolysat désacidifié a un rapport de HCl à saccharides totaux de ≤ 0,03 en poids.

4. Procédé selon l'une quelconque des revendications précédentes, dans lequel l'hydrolysat désacidifié a des oligosaccharides supérieurs en un rapport à saccharides totaux ≤ 0,2.

5. Procédé selon l'une quelconque des revendications précédentes, dans lequel un rapport d'au moins un dudit di-glucose alpha-lié et dudit di-glucose bêta-lié relativement aux saccharides totaux est (i) > 0,01 dans l'hydrolysat désacidifié ou (ii) > 0,03 dans l'hydrolysat désacidifié.

6. Procédé selon l'une quelconque des revendications précédentes, dans lequel ledit di-glucose alpha-lié inclut au moins un de : maltose, isomaltose et tréhalose.

7. Procédé selon l'une quelconque des revendications précédentes, dans lequel ledit di-glucose bêta-lié inclut au moins un de : gentiobiose, sophorose et cellobiose.

8. Procédé selon l'une quelconque des revendications précédentes, dans lequel le solvant S1 est : hexanol ou hexanol 2-éthylique.

9. Procédé selon l'une quelconque des revendications précédentes, qui comprend l'enlèvement de HCl résiduel dans la solution de sucre appauvrie en HCl par (ii-a) séparation chromatographique.

10. Procédé selon l'une quelconque des revendications précédentes, où ladite étape d'hydrolyse (a) forme ledit hydrolysat et une lignine résiduelle, et ledit procédé comprend en outre :
(b) la séparation dudit hydrolysat à partir de ladite lignine résiduelle avant ladite étape de désacidification (c).

11. Procédé selon l'une quelconque des revendications précédentes, qui comprend en outre
(d) l'hydrolyse enzymatique de l'hydrolysat désacidifié avec un enzyme capable d'hydrolyse catalysante d'alpha-liaisons dans le mélange pour qu'au moins 10 % des disaccharides soient convertis en monosaccharides.

12. Procédé selon l'une quelconque des revendications précédentes, qui comprend en outre
(e) la fourniture d'un fermenteur ; et
(f) la fermentation d'un milieu comprenant l'hydrolysat désacidifié dans ledit fermenteur pour produire un produit de fermentation.

13. Procédé selon la revendication 12, dans lequel ledit procédé comprend en outre :
(gl) le traitement dudit produit de fermentation pour produire un produit de consommation sélectionné parmi le groupe constitué de : détergent, produits à base de polyéthylène, produits à base de polypropylène, produits à base de polyoléfine, produits à base d'acide polylactique (polylactide), produits à base de polyhydroxyalkanoate et produits à base de polyacrylique ; ou
(g2) la production d'un produit de consommation comprenant le produit de fermentation, dans lequel ledit produit de fermentation est au moins un sélectionné parmi : acides carboxyliques et gras, acides dicarboxyliques, acides hydroxylcarboxylique, acides di-carboxyliques d'hydroxyle, acides gras d'hydroxyle, méthylglyoxal, mono-, di-, ou poly-alcools, alkanes, alcènes, aromatiques, aldéhydes, cétones, esters, biopolymères, protéines, peptides, acides aminés, vitamines, antibiotiques, et produits pharmaceutiques ; ou
(g3) la production d'un produit de consommation comprenant le produit de fermentation, dans lequel ledit produit de consommation est : essence enrichie en éthanol, carburéacteur, ou biodiesel.
